# EUROPEAN PATENT APPLICATION

(11) **EP 4 618 091 A1**
(43) Date of publication of application: **17.09.2025**
(21) Application number: 24162593.8
(22) Date of filing: 11.03.2024
(51) Int. Cl.: G16H 20/40, G16H 40/63

(54) **METHOD FOR ASSISTING A USER AND MEDICAL SYSTEM**

(71) Applicant: Fresenius Medical Care Deutschland GmbH, 61352 Bad Homburg (DE)
(72) Inventor: Kipp, Sabine, 61352 Bad Homburg (DE); Heinze, Marcel, 61352 Bad Homburg (DE); Jakobi, Tim, 61352 Bad Homburg (DE); Witzel, Stephan, 61352 Bad Homburg (DE)
(74) Representative: Schwarz, Claudia

(57) **Abstract**

The present invention relates to an assistance of a user when dealing with a medical device such as a dialysis device. For this purpose, it is proposed to provide the user with assistance by means of an augmented reality device. The assistance may be provided by analysing a current situation and the needs of the user. An operational setting of the dialysis device is detected for providing automated assistance to a user. In further embodiments, the user can be assisted by an expert, wherein the user can be assisted for a full or partial automated selection of an appropriate expert.

## Description

The present invention relates to a method for providing assistance for operating a medical device and in particular on a dialysis device by means of an augmented reality device. The present invention further relates to a medical system comprising a medical device and an augmented reality device. Further, the invention relates to an augmented reality device. In particular, the present invention relates to providing assistance for operating a dialysis a device or when dealing with the same.

Even though the present invention is described in the following by way of example with reference to a dialysis device, the general concept may be also applicable to other devices, in particular other medical devices, especially blood treatment devices.

More and more medical devices are becoming increasingly complex. An example of such a complex medical device is a dialysis device. Such medical devices require complex operations and configurations which have to be performed by the user. Further, numerous accessories and consumables should to be taken into account when operating medical devices.

Since improper operations could be dangerous for a patient, it is of great importance that a user, e.g. a nurse or doctor, is familiar with the device. In case of uncertainties, it is desirable that the user can be guided fast, safely and reliably through the tasks.

At present, the user has to read the manual of the respective device in order to learn to operate it. Further, even if the user may be trained by one or more human trainers, for this training purpose, the user and the trainer must be present at the same location. In case of unusual tasks or a malfunction of the device, the user may easily become stressed or nervous. This may lead to situations in which the user possibly could not react quickly enough or even would make mistakes.

In view of this, there is a need for a system providing improved assistance of the user when operating a complex medical device such as a dialysis device. In particular, there is a need for a method and a system for controlling components which could assist the user in operating the medical device.

### Summary

The present invention provides a method for assisting a user on a dialysis device and a medical system with the features of the independent claims. Further advantageous embodiments are subject matter of the dependent claims.

According to a first aspect, a method for technically providing assistance when operating a medical device is provided and in particular on a dialysis device. The method comprises capturing data. The captured data may be related with the medical device or may be related with activities of the user, when interacting with the medical device. The data may be captured by at least one sensor component. The method further comprises identifying an operational setting of the medical device. The operational setting may be determined based on the data captured by the at least one sensor component. Further, the method comprises determining a task to be executed by the user on the medical device. The method further comprises monitoring activities of the user. The activities of the user may be monitored based on the data captured by the at least one sensor component. Further, the method comprises determining an assistance scheme for providing assistance to the user. The method may further comprise to determine an assistance according to the determined assistance scheme. The method further comprises to provide assistance according to the determined assistance scheme. The assistance scheme and the assistance itself may be determined specifically for the user for carrying out the determined task. In particular, the determination of the assistance scheme may consider the identified operational setting of the medical device, the determined task to be executed and the monitored activities of the user. Providing the assistance to the user according to the determined assistance scheme comprises the output of indications on an output component of the AR; device. The term "assisting a user" refers to providing assistance by technical means, in particular by control of the AR device.

The first aspect relates to the claimed method. Features, advantages or alternative embodiments which are described or claimed with respect to the method can be also transferred or assigned to the other claimed objects (e.g. the system, the augmented realtiy device, computer program) and vice versa. In other words, the device can be improved with features described or claimed in the context of the method and vice versa. In this case, the functional features of the method are embodied by structural units of the apparatus or device or system and vice versa, respectively. Generally, in computer science a software implementation and a corresponding hardware implementation (e.g. as an embedded system) are equivalent. Thus, for example, a method step for "storing" data may be performed with a storage unit and respective instructions to write data into the storage. For the sake of avoiding redundancy, although the device may also be used in the alternative embodiments described with reference to the method, these embodiments are not explicitly described again for the device. In principle, the claimed device or apparatus claim is adapted to carry out the claimed method.

According to a further aspect, a medical system is provided. The medical system is configured for providing assistance on the AR device, in particular when for operating a medical device. The medical system is configured to execute the method or any embodiments or alternatives as mentioned above. The medical system comprises a medical device, in particular a dialysis device, an augmented reality device, a control device and a sensor component. The sensor component is configured to capture data. The captured data may relate to the medical device and/or to activities of the user, when interacting or operating with the medical device. The control device may be a control device integrated in the medical device. For example, the control device may be realised by components of the medical device. Alternatively, the control device may be also realised by a separate device which is arranged outside the medical device. Furthermore, the control device may be also realised by a combination of components included in the medical device and components outside the medical device. The control device is configured to identify an operational setting of the medical device. In particular, the operational setting may be identified based on the data captured by at least one sensor component. The control device may be further configured to determine or to receive a task to be executed by the user on the medical device. Further, the control device is configured to monitor activities of the user. In particular, the activities may be monitored based on the data captured by at the least one sensor component. The control device is further configured to determine an assistance scheme for providing assistance to the user, in particular an assistance scheme for carrying out the determined task. The assistance scheme may be determined based on the identified operational setting of the medical device, the determined task to be executed and the monitored activities of the user. The control device may be configured to determine the assistance. The control device may further be configured for providing the (in particular determined) assistance on the AR device (related to the user) according to the determined assistance scheme, comprising control of the output of indications on an output component of the AR device.

In an embodiment, more than one AR device is used, in particular a first AR device to be used by the user of the dialysis device and a second AR device, which is used by the human expert. The assistance may be provided on the first and on the second device, so to simulate the real operating conditions of the dialysis device as much as possible for the assisting human expert.

The assistance scheme may define the kind, form and/or manner, the assistance should be provided. For example, the assistance scheme may define when, how long (time interval), how often (number of iterations) and/or in what form (e.g. optical representations to be output on the augmented reality device, and/or acoustic format and/or in a haptic form) the assistance is provided. Also, the skill level of the expert for providing the assistance may be selected.

The present invention is based on the finding that operating a complex medical device such as a dialysis device is a great demand for the users. A user has to study the manuals and has to be extensively trained to use such a device. In particular, there may be numerous exceptional situations which have to be handled by the user. Since some failures of operating the device may lead to a health risk, the user has to operate to device very carefully in order to avoid such failures. This may be a real challenge, especially under time pressure.

The present invention therefore takes into account this finding and aims to provide an approach for assisting and/or teaching a user when operating a complex medical device such as a dialysis device. In particular, the present invention aims to provide a method and a system which could assist the user under real circumstances when operating the device. For this purpose, the user can be provided with multiple kinds of assistance by means of an augmented reality, AR, device.

An AR device in the context of the present invention may be understood any as kind of device providing AR features. For example, such an AR device may be an AR or mixed reality, XR, headset such as, e.g., Microsoft HoloLense. However, any other kind of AR or XR device may be possible, too. The AR device may be also any other kind of device providing AR features. For example, a smart phone, tablet computer or any other kind of device with a camera and a display may be used as an AR device. In general, any kind of device which has capabilities for providing an overlay of a real scene with additional (artificial) elements may be used as AR device. "Augmented" in the present context refers to the fact, that the real or physical scene may be augmented or enriched with additional information, which may be provided as virtual indications.

An output component in the context of the present invention may be any kind of component for outputting optical, haptic and/or acoustic indications. The output component may be implemented, at least in part, by the above-mentioned AR device. In particular, the output component may be part of the AR device. For example, the output component may comprise a display for providing optical information or indications. In particular, the output component may comprise a display device for overlaying a real scene with additional virtual elements/indications. For this purpose, a semi-transparent display may be used for providing additional indications on a really scene to which the display is pointed. Alternatively, s scene may be captured by a camera, and the image data from the camera may be overlaid with additional virtual indications on the display. Alternatively or in addition the output component may be implemented on another device, in particular a mobile device and/or the medical device although it is referred to as "output component of the AR device". In the latter case the output component may be outsourced or transferred from the AR device to an AR-external device.

Generally, an indication may be any kind of information, provided or represented on the output component. An indication may be provided in different forms, in particular in an optical, acoustic, haptic or other form. The output component is selected accordingly (optical output, loudspeaker output, vibrational output etc.)

Optical indications may be any kind of graphical representation which can be used in the context of operating the medical device. For example, an optical indication may be a graphical element, an icon, an image (e.g. a photo or a drawing of a component of the medical device), a line, arrow, box, circle or any other geometric element, a text box with text and/or other information, a video sequence, and avatar, etc. The graphical representation may be a static graphical representation and/or a dynamic representation such as a video sequence, a moving or turning graphical element, etc.

Acoustic indications may be, for example, a sound such as a beep or a warning tone, a sound sequence, melody or spoken text. The spoken text may be an artificially generated text message or a text messages recorded from the human being. However, any other kind of acoustic indication may be possible, too.

A haptic indication may be, for example, a vibration, shaking and/or any other haptic output.

A sensor component in the context of the present invention may be any kind of input component, in particular any kind of component for sensing an environmental property and/or for receiving user input, e.g., like a user interface, UI, graphical user interface, GUI etc. For example, the sensor component may comprise optical sensor elements such as at least one camera, at least one laser scanner or the like. Alternatively or in addition, the sensor component may also comprise an acoustic sensor element such as a microphone, which can be used, for example, for recording a voice command of the user. Alternatively or in addition, the sensor component may further comprise sensor elements such as a gyroscope, an acceleration sensor (for one or more dimensions) and/or a distance sensor or any other kind of sensor for sensing a position, pose and/or movement of the user. Alternatively or in addition, the sensor element may also comprise one or more communication modules. For example, such a communication module may be a Wl Fl-interface, a Bluetooth-interface, an RFID-reader or any other wireless or wired communication module. However, it is understood, that any other kind of the sensor element may be also comprised the by the sensor component. The sensor component or parts thereof may be part of the augmented reality device. Alternatively or in addition the sensor component may be provided as external device, in particular external to the medical device and/or external to the augmented reality device. The sensor component may be a part of a mobile device (e.g. smartphone, tablet etc.).

One or more optical sensor elements of the sensor component may be used for capturing images in the environment of the user. In particular, an optical sensor may capture image data of the environment to which the user is currently looking. The image data captured by an optical sensor may be used, for example, for identifying a medical device which is currently operated by the user. Especially, the image data may be used for identifying attachments and/or accessories which are mounted on the medical device or which are available in the environment of the medical device. For example, the medical device, the attachments or accessories may be identified based on a pattern recognition approach for identifying characteristic elements of the respective elements. It may be also possible to scan a code, e.g. a QR code or the like in order to identify an element. However, any other approach for identifying the device or a further component may be also possible.

The data captured from the sensor component, e.g., captured by means of one or more optical sensor(s) may be also used, for example, for identifying an operational setting of the medical device. The operational setting may refer to a current configuration and/or state of the medical device and/or properties of the medical device, like e.g. position of device, device parts and/or disposables and/or accessories, to be used for device operation. For example, image data may be analysed in order to identify a current setting of a control element and/or to analyse an output of the display on the medical device.

Further, data from the sensor component may be used for monitoring and/or evaluating activities of the user. For example, an optical sensor may capture image data of a hand of the user or parts thereof (fingers etc.). Such data may be used in order to analyse a current operation, for example a movement towards a control element, an operation for attaching or removing accessories, an action in connection with a patient, or any other manual operation of the user.

The data, captured by means of the sensor component may relate to 1. the medical device and/or related parts and/or operating conditions thereof and/or to 2. activities of the user, when interacting with or operating the medical device.

Image data from one or more optical sensors may be also used, for example, in order to evaluate an available space surrounding the user and/or the medical device. For example, dimensions of the room in which the user and the medical device are located may be determined. Accordingly, it may be possible to consider spatial constraints in case of limited space due to small rooms or further devices located in the environment.

Further, it may be also possible, to determine, based on image data and/or other available data, a spatial location of the user and/or the medical device as an operational setting. For example, it may be possible to determine whether the user and the medical device are located at a hospital, a medical facility, at the patient's home, etc. Furthermore, it may be also possible to determine additional infrastructure which would be available and/or which would be needed for operating the medical device as operational setting.

Interfaces or communication modules may be used for any kind of communication purpose. The communication may be performed by a wireless or wired communication. For example, a communication may be established with the medical device. Such a communication with the medical device may be used, for example, in order to determine a type of the medical device, determine a current configuration of the medical device, determine a status of the medical device, obtain an error messages from the medical device, etc. Further, communications may be established to one or more databases. For example, is possible to refer to a database in order to obtain additional information of a medical device, obtain information of a user, e.g. preferences of the user, a training status, etc., obtain patient information, obtain operational information in connection with the medical device, receive updates, e.g. from a manufacturer, receive supplementary information for assisting the user, etc. However, it is understood, that any other kind of information may be also obtained from one or more databases.

Interfaces or communication modules may be also used to establish any kind of communication with an artificial or human expert. In case of an artificial expert, information for assisting the user may be obtained from a local or remote computing device. In case of a human expert, a communication channel may be established with such a human expert in order to obtain further assistance from the expert and/or to provide the expert with information of the medical device and/or the current user. In such a case, the human expert may be located either close to the medical device and the user. For example, the expert and the user may be located in the same room. Alternatively, the expert may be located spatially separated in another room, another building, another city, country or continent. In case that the human expert is located spatially separated, the remote expert may be provided with any kind of appropriate information such as configuration of the medical device, status/failure messages of the medical device, attached or available accessories, preferences of the user, etc. The human expert may be also provided with further real-time information such as images captured by a camera, a voice communication with the user, etc. The human expert may be provided with such information, for example by any kind of output devices such as a loudspeaker, a headset, a smartphone or tablet computer, a monitor, an VR, AR or XR device, etc.

Alternatively or in addition, the human expert may use a second AR device where the same or a similar indications are provided compared to the output of the AR device, the user is using who has requested assistance. With this, the output on the AR device, the user is using and the second AR device the human expert is using are comparable in order to be able to simulate the situation of the user at the medical device as realistic as possible.

A task to be executed in the context of the present invention may be any kind of operation or action in connection with the related medical device. A task may require at least one activity and in particular a set or sequence of activities to be carried out manually by the user or automatically via instruction.

Such a task may be, for example, preparing the medical device for a desired task/job (e.g. setup of the medical device, in particular dialysis machine, in particular prior to commissioning of the medical device). For this purpose, a user may have to mount one or more attachments or accessories on the medical device, insert or connect one or more consumables, make specific settings on the medical device or perform any other required operation. If necessary, particular data of a patient have to be taken into account.

A task may be, for example, monitoring the medical device(s) during operation. For this purpose, the user may have to monitor the status messages of the medical device, monitor a filling level of the consumables, and/or monitor flow rates, etc. In case that one of the parameters is out of tolerances, the user has to react on this with appropriate activities in order to ensure safe operation.

In another example, a task may be, for example, reacting in case of a failure or error message. For example, if the medical device detects a failure or problem, the user has to take the necessary steps (activities) in order to remove the reason for such a problem. However, it is understood, that a task may comprise any other kind of operation in connection with the related medical device, such as maintenance, normal operation, testing, or training.

A task may be specified or selected, for example, by the user, for instance by user input. Additionally or alternatively, a task may be determined fully or at least partly automatically. For this purpose, further information such as data from the medical device, from a patient database or other sources/databases may be taken into account in order to determine a desired task. Further to this, it may be also possible that another person, for example a trainer or instructor, may select a task which should be executed by the user.

An assistance scheme defines a scheme or pattern for providing the assistance. An assistance scheme may define how, where (in particular, where or on which device the output component is implemented and instructed for providing the indications) and/or in which form the assistance in form of the output indications is to be provided technically, in particular with respect to the determination of the appropriate output component, time intervals, duration, output format (optical, acoustical, and/or haptical etc.), and/or intensity (brightness for optical indications or volume for acoustical indications etc). The assistance scheme may comprise or refer to optical, acoustic or haptic indications. For example, the assistance scheme may refer to or comprise haptic indications such as a shaking or vibration in order to draw the user's attention. The assistance scheme may refer to or comprise acoustic indications such as a beep, warning tone, melody, any other kind of sound or even a spoken text message. The assistance scheme may further refer to or comprise optical indications such as arrows, lines, circles, boxes, icons, images, text messages, video sequences, etc., in order to guide a user through the related task. The indications may be provided on the output component of the AR device. However, it is understood, that any other kind of assistance scheme may be used in order to provide appropriate assistance to a user for carrying out the desired task.

In the context of the present invention assistance may be any kind of assistance which could be used for assisting in the user in operating the medical device. For example, such an assistance may comprise information for guiding the user through a desired task (with a sequence of timely ordered activities), e.g. configuring the medical device, attaching accessories, adapting the medical device to a particular patient, operating in the medical device, performing the steps in response to a waring or error message, refilling of consumables, performing maintenance operations, etc.

The method according to the present invention may comprise to calculate or determine algorithmically an assistance. The algorithmic determination of the assistance may define the content of the assistance in form of e.g., instructional messages or indications. The determined assistance is provided in accordance with the determined assistance scheme. The assistance is provided on the output component, which may be implemented at least partially on the AR device and optionally on related devices, such as mobile devices assigned to the user an/or the medical device and/or parts thereof. The assistance may comprise output of indications, for example, a description for a single step or a sequence of steps within an activity which should be performed by the user in order to execute the desired task. The assistance may comprise, for instance, information for an appropriate setting of the medical device, information for required additional components or consumables, or the like. The assistance may also comprise, for example, indications to one or more specific positions at the medical device or related components, indications of how to operate a control element of the medical device, specifications how to handle or grasp a particular component, or any other information related with the desired task to be executed.

Assistance according to the assistance scheme can be provided to the user in any appropriate manner. In particular, the assistance may be provided to the user by an appropriate output component. The output component may comprise, for example, optic devices such as one or more displays for providing optical indications. The output component may also comprise acoustic elements such as a loudspeaker, a headphone or the like in order to provide acoustic information. Further to this, the output component may comprise any other kind of appropriate output element, example for providing haptic output.

The output component may be comprised, at least in part, by an AR device. For example, optical indications may be provided by means of a display of the AR device. Further, acoustic indications may be provided by means of an acoustic output element of the AR device. However, it may be also possible that the AR device is connected to one or more further output elements so that at least part of the desired indications could be also provided by such external output elements not comprised by the AR device itself, e.g., on the medical device and/or on a mobile device, which may be registered in the system.

The operations for identifying an operational setting of the dialysis device, determining a task to be executed by the user, monitoring activities of the user and determining assistance of the user may be performed, for example, by an appropriate control device. Such a control device may be realised, for example, by a processor and a memory communicatively coupled to the processor. The memory may be configured to store program code which can be read by the processor. The program code may cause, when executed by the processor, the control device to perform appropriate operations, in particular operation for executing at least part of the method for assisting the user on the medical device. The control device may comprise further components such as interfaces for receiving data and/or outputting a data. In particular, the control device may comprise interfaces for any kind of wireless or wired communication. Furthermore, the control device may comprise elements such as any kind of appropriate input and/or output devices, for example a user interface for interacting with the user. However, it is understood, that the control device may comprise also any other kind of appropriate components, if necessary.

Activities of the user may refer to any actions the user is executing with respect to the medical device, like e.g., handling, operating or moving the device or parts or accessories thereof, taking actions which cause an effect of the medical device, like clicking buttons on the device or on a user interface.

The control device may be a control device which is arranged separate to the medical device, in particular separate to the dialysis device. However, the control device may be also integrated, at least in part, within the medical device. Especially, the control device may be realised, for example, by appropriate components of the dialysis device. In this way, the control device may be fully or at least partially implemented by the component of the medical device.

In a possible embodiment, the step of capturing data related with the medical device may comprise capturing an image, scanning an optical label and/or an RF tag of the medical device and/or an accessory component of the medical device by the sensor component. Further, the method, and in particular identifying an operational setting of the medical device, may comprise identifying the medical device, detecting at least one accessory component of the medical device, and/or determining a spatial position and/or a spatial orientation of the medical device. For example, image data may be captured by a camera of an AR device. The image data may be processed, for example, based on an appropriate image processing algorithm, in order to identify a particular medical device, classify a type of the medical device, determine a configuration of the medical device, e.g. identify accessory or attachments of the medical device, etc. Furthermore, it may be also possible, for example, to identify an optical code, for example a quick response (QR) code on the medical device or an accessory of the medical device. Accordingly, the medical device or an accessory may be identified based on such a code. However, it may be also possible to use any other kind of appropriate device for scanning and/or identifying the medical device or an attachment accessory of the medical device. For example, a barcode reader, an NFC reader or the like may be used in order to read an appropriate tag on the medical device or an accessory. In this way, the medical device and its configuration can be easily determined in an automated manner.

In a possible embodiment, the method may comprise a step of obtaining at least one related reference operation for the determined task. The related reference operation may be obtained, for example, from a reference database. Accordingly, the assistance scheme and/or the assistance may be determined based on a comparison between the monitored activities of the user and a related reference operation obtained from the reference database. Related reference operations may be stored, for example, in any appropriate manner, e.g. a reference database. The reference database may be provided locally in the medical device. However, it may be also possible to obtain the related reference operations from a remote device such as a remote server, especially a cloud or the like. The reference operations may specify, for example one or more operations which should be executed by a user in order to fulfil a desired task. The reference operations may comprise, for example, information relating to manual operations of the user such as "connecting a particular element on a related terminal of the device", "inserting a particular amount of a consumable into a specific reservoir of the device", "entering specific settings on an input terminal of the device", "verifying whether a particular component has been securely mounted", etc. The reference operation may also comprise information relating to a required order for executing operations. In this way, it is possible to specify all necessary operations which have to be executed by the user to perform an operation correctly and completely.

In a possible embodiment, the task to be executed by the user may comprise a task for configuring or operating the medical device, for monitoring the medical device during operation, and/or a task for maintenance of the medical device and/or a task for a repair or service of the medical device. However, any other appropriate task which could be executed in connection with the medical device may be possible, too. Accordingly, each action or activity which has to be performed with respect to the medical device can be part of a task. A task may be a general operation in connection with a medical device.

However, it may be also possible that a task relates to a particular desired goal which should be achieved by using the medical device, for example a particular treatment of a patient. Accordingly, a task may be also patient-related. For this purpose, it is possible to refer to patient-related data. For example, such a patient-related data may be read from a storage device, for example a patient-database on a remote server or a cloud. Furthermore, a task may be also user specific (user, who is operating the medical device). For example, depending on the knowledge or capability of a current user, different types of a task may be selected. For example, a skill level of the user may be taken into account when selecting an appropriate task. For this purpose, user specific data may be received, for example, from a user-specific database on a remote server or cloud.

Additionally or alternatively, the user may also enter user specific information by means of an appropriate input terminal, in particular an input terminal of the medical device. A task may also take into account a current configuration of the medical device/dialysis device. For example, it may be possible to receive device specific information by means of a wired or wireless interface from the medical device. For example, information of connected or available accessories, may be received and taken into account for selecting an appropriate task to be executed. Furthermore, a particular setting, for example a fill level of one or more consumables may be also taken into account, when determining the task. For example, a task may refer to a refill of a consumable if a fill level is below a predetermined threshold.

Further, status messages and/or failure messages may be received from the medical device. Accordingly, a task may be determined for resolving a current problem such as a failure or the like. For example, a task may relate to an operation for cleaning a filter if a flow rate or pressure level deviates from a predetermined range. A task may also relate to a maintenance operation, which shall be executed in case that a specific failure occurs, or a point of time for a specific maintenance interval is reached.

In a possible embodiment, the assistance scheme and/or the assistance may be determined based on a preset or predetermined skill level of the user. Alternatively or in addition, the assistance scheme and/or the assistance may be determined based on one or more rules. In particular, such a rule may be prestored and/or obtained from a rule database. The rule database may be any kind local or remote storage, for example a remote server or a cloud. The skill level may be determined, for example, based on any kind of user input. For this purpose, the user may enter his skill level by means of an appropriate input device, for example a user interface. Alternatively or additionally, the skill level may be also determined at least in part in an automated manner. For this purpose, the operation of the user may be monitored. Further, any kind of appropriate rule for determining the assistance scheme may be possible. Such a rule may specify, for example, relationships between the configuration and/or features of the medical device, the task to be executed, information in connection with a patient and/or the user of the medical device.

In a possible embodiment, the method may further comprise a step of computing an evaluation of a skill level of the user. The skill level of the user may be computed based on the monitored activities of the user and/or the related reference operation for the determined task. For this purpose, the activities of the user may be monitored/sensed by means of any kind of appropriate device. For example, sensor components, such as camera, Lidar sensors, motion sensors, ultrasound-based sensors, and/or acceleration sensors, etc. may be received and processed in order to monitor activities or operations of the user. The monitored activities may be compared, for example, with related reference operations in order to determine deviations and/or identify failures during the operation or activities of the users. Thus, the skill level of the user may be determined based on a degree of deviation and/or a number of failures. This degree of deviation may be compared with a preset threshold and in case the threshold is not met, a rule may determine that the assistance is to be provided or that assistance is to be strengthened.

Furthermore, a time period for executing a specific task/operation may be taken into account for determining the skill level of the user. For example, the real time period may be compared with one or more reference values in order to assess the skill level of the user. However, any other scheme for evaluating the trading level of the user and determining a skill level of the user may be possible, too.

In a possible embodiment, the assistance scheme and/or the assistance may be determined depending on a difference between the monitored activities of the user and the related reference operation. In this way, the number of indications for assisting the user can be limited. Accordingly, an amount of distraction the user can be minimised during the operation. For example, the indications may be provided on the output component only, if a difference between the monitored activities of the user and a related reference operation is determined. In this way, the user may operate the medical device without or only with a minimum amount of assistance as long as the operation of the user is in line with related reference operations. In case that the operations of the user deviate from the reference operations, an appropriate indication may be provided to the user so that the user can recognise a possible failure in good time. Furthermore, it may be possible to adapt the indication depending on a degree of deviation between the current operation of the user and the related reference operations. For example, only a small indication such as a line, arrow of another symbol may be shown on a display in case of a small deviation between the operation of the user and the reference operation. In case of larger deviations, the indication may comprise a larger graphical element, a flashing graphical element, or an output of an acoustic signal or a haptic indication.

The method according to one aspect, mentioned above, may comprise using a large language model, LLM. In particular, the LLM may be used to provide assistance to the user, e.g., in the form of calculating the indications. The LLM may be trained for acquiring the abilities, mentioned before by learning statistical relationships from text documents or other information sources or files during a computationally intensive self-supervised and semi-supervised training process. The LLM may be built with a decoder-only transformer-based architecture. Alternatively or in addition other architectures may be used, such as recurrent neural network variants and Mamba (a state space model). In particular, the LLM may be used, to get an answer to a question provided in relation to the dialysis device. The answer may be provided on the output component of the AR device or on an output component of the dialysis device or of a user's (e.g., mobile) device. The user can ask the system by using the LLM, for example, which part of the machine is currently in front of him, where to place a disposable, where a certain part of the machine (e.g. the blood pump) is located, or ask for help with the next step. The system recognises the situation in the room with the help of AR sensors and/or the at least sensor component, evaluates the user's verbal interaction of the user accordingly using the LLM and provides corresponding information such as AR highlighting, text, arrows and/or prepared videos and/or graphics to answer the verbal user enquiry. This can be done in any language if the LLM has the appropriate language capabilities. The system may respond automatically in the language of the request (enquiry).

In a possible embodiment, the method may comprise a step of providing real-time support for operating the device. In particular, the support may be provided from an artificial expert. Additionally or alternatively, support may be provided from a human expert. The real-time support may be provided on an output component, in particular the output component which is used for providing assistance by means of indications according to the determined assistance scheme. In particular, the real-time support may be provided on an output component of the AR device.

For providing support, in particular real-time support, from an artificial expert, any kind of appropriate technical system may be used. For example, a computing device may generate data which are provided to a user to achieve a desired result. For this purpose, the support may comprise data for providing any kind of indications such as text or graphical elements on a display, video sequences, sound signals or spoken text messages, etc. The artificial support may be generated, for example, based on any data in connection with the current operation of the user. For example, the system for generating artificial assistance may receive information from the medical device or one or more further sensor components, which monitor, for example, the operations of the user. Accordingly, such a system of an artificial expert can provide appropriate assistance depending on a current situation.

Additionally or alternatively, the user may be provided with real-time support from a human expert, i.e. a real person. For this purpose, any kind of communication link between the human expert and the user may be established. For example, a user may be provided with a voice signal from the human expert. Furthermore, the user may be provided with graphical information such as a video from the human expert and/or a video from a further (same or similar) medical device which is operated by the human expert. However, any other manner for providing real-time support may be possible, too.

In a possible embodiment, the method may comprise a step of receiving a user request for requesting (the provision of) real-time support. Thus, the user request reflects the user's intention and instruction to obtain real-time support. In particular, the user request may be received by a sensor component, especially the sensor component which is used for monitoring the activities of the user. The user request may be received in any appropriate manner. For example, the activities of user may be monitored in order to identify a specific gesture of the user such as a predetermined movement of a finger or a hand of the user. However, any other kind of gesture may be possible, too. The user request may be also triggered based on an acoustic input, e.g. based on speech recognition or the like. Accordingly, assistance from the artificial or human expert can be initiated and controlled based on the received user request. The providing of the real-time support may be controlled based on parameters of the received user request. Parameters may refer to a timing of the real-time support, a duration, and/or an intensity, a determination of the output component (i.e. where to provide the indications of the assistance). This has the technical advantage, that unnecessary real-time support can be avoided.

In a possible embodiment, the expert, in particular a human expert, may be identified by means of an identification algorithm, which may be based on predetermined selection rules. The identification algorithm may be configured to select or determine a human expert out of a set of human experts, generally registered in the system. The identification algorithm may process personal information. Additionally or alternatively, the identification algorithm may be based on prestorable personal information related to human experts. For example, personal information of one or more human experts may be stored in an appropriate memory such as a database. The database may be a local or remote database, for example a database in a cloud. Information relating to a human expert may specify, for example, specific expertise such as knowledge in connection with one or more types of medical devices, expertise in connection with specific treatment methods or diseases, but also language skills, a time schedule specifying the availability of an expert, geographical information, where the expert is situated, references in connection with specific users or classes of user, or any other feature which could be appropriate for selecting a specific expert.

Alternatively or in addition, the expert, in particular the human expert is assigned or matched to a user's request or to a medical device, the user is operating by means of an assignment algorithm. The assignment algorithm may read-in and process personal information relating to the human expert and/or the identified operational setting of the medical device and/or the determined task and/or the monitored activities or the user, performed in order to calculate an assignment of the human expert to the request for assistance. The request of assistance may be received on a sensor component, like a UI, which may be implemented on the medical device or at least in part on the AR device.

In a possible embodiment, the method may comprise determining personal information for a human expert. In particular, the personal information may be determined by receiving input from the human expert, analysing data, in particular scheduling data, from an electronic device of the human expert, detecting a presence of the human expert at a predetermined spatial location, receiving a rating of the human expert according to an input of the user or any other manner for an automated determination of personal information of the human expert. For example, an availability of a human expert may be determined by detecting the presence of a user in a specific room or building, analysing an electronic calendar of the expert, etc. For example, the determination may be performed by establishing a connection to a device or server storing personal information related to the expert and receiving information from this device or server. The information of an expert may be also received by means of an input terminal or user interface wherein the expert enters his personal information manually. Further, it may be also possible to specify further information of an expert by rating received from one or more users. For example, a user may provide such a rating after the assistance by the respective human expert has been finished. The information about least one human expert may comprise skills, preferences, scheduling data, positional data, a rating and/or further data related with the respective human expert. The method may further comprise a step of storing the determined personal information in a database. For example, the personal information may be stored in a local or remote storage device, for example a remote server or cloud.

Furthermore, the method may comprise a step of determining demands of a user for operating the medical device. Accordingly, the method may further comprise a step of identifying a human expert based on a matching between information stored in the database and the demands of the user. In this way, an appropriate human expert can be selected in an automated manner within a very short period of time. This can be very advantageous for time-critical operations in connection with the operation of a medical device.

In a possible embodiment, the demands of the user may be determined based on user input. In particular, user input may be received by the sensor component and/or input data may be received from the medical device. Additionally or alternatively patient information may be received. In particular, patient data may be obtained from a patient database, in particular a remote database such as a remote server or cloud.

In a possible embodiment, the method may comprise a step of storing operational activities of a user. The activities of the user may be determined in any appropriate manner. For example, one or more sensor components for monitoring the user activities may be received and processed in order to determine the respective user activities. For example, image data captured by one or more cameras may be processed, data from a data interface of the medical device may be received, or any other approach for identifying activities of the user may be performed. The activities of the user may be stored in an appropriate manner locally or in a remote storage device. For example, an appropriate coding scheme may be used for characterising the individual activities of the user. If, for example, two or more possibilities exist for solving a specific task or problem, it can be stored in the respective database which solution is preferred by the respective user. Accordingly, an appropriate human expert can be identified based on previously stored operational activities of the user. In this way, the user can be assisted by a matching human expert which fits to the preferences of the user.

In a possible embodiment, the human expert is selected based on a machine learning module, which is configured to implement an artificial intelligence approach. The machine learning module may be implemented as neural network architecture, in particular as a DNN, deep neural network. For example, a neural network may be used in order to analyse any kind of available information from the human expert and/or from the user, operating the medical device. Furthermore, the neural network may be also used for analysing preferences of the user and/or a current situation or demand of the medical device which will be operated by the user. In particular, the neural network may be trained with training data, comprising the operational setting of the medical device, the monitored activities of the user and an associated assistance. The neural network is trained to provide an assistance for a respective request of assistance, i.e. for new, unseen input data, including the operational setting of the medical device etc as mentioned above.

In another aspect an AR device is provided for use in a medical system as described above.

In the following detailed description of the figures, non-limiting examples of embodiments with their features and further advantages are discussed with reference to the drawing.

### Brief description of the figures

Fig.1: shows a schematic illustration of a medical system according to an embodiment;
Fig. 2: is a schematic illustration of a user with an AR device according to an embodiment;
Fig. 3: depicts a schematic illustration of a user with another AR device according to an embodiment;
Fig. 4: shows a schematic illustration of a flow diagram underlying a method according to an embodiment; and
Fig. 5: is a schematic illustration of an AR scene is generated by an AR device according to an embodiment.

### Detailed description of the figures

In the following, the invention is described in more detail with reference to embodiments in connection with the figures.

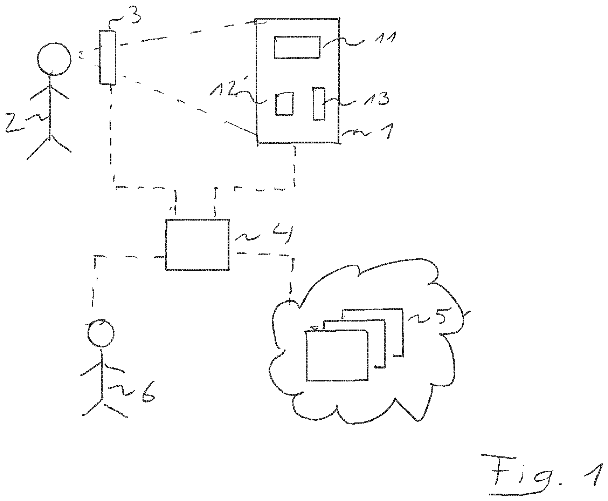

**Figure 1** shows a schematic illustration of a configuration with a medical system according to an embodiment. The medical system comprises a medical device 1, in particular a dialysis device or another blood treatment device. The medical device 1 may be operated by a user 2.

Blood treatment devices, in particular dialysis devices, are medical devices in which the blood of a patient is usually treated extracorporeally. For example, drugs may be added to the blood or blood components removed from it, blood may be heated or cooled. These processes are often monitored by sensors. Blood treatment devices have a variety of means for these purposes, such as pumps, valves, sensors, clamps or the like. Furthermore, medical disposables are often used for blood treatment. Such disposable articles are usually discarded after treatment for reasons of hygiene. Medical disposables, or so-called disposables, are, for example, tube sets for administering medical fluids such as blood or medicaments, filters, such as adsorber filters or dialysis filters, sachets for storing medicaments, and the like. These disposables are usually inserted by a user in the blood treatment device, or connected to the same and removed after treatment from the blood treatment device or separated therefrom.

In the example of figure 1, a medical device 1 (in short: device) is illustrated, wherein the device 1 comprises an input/output component 11, and at least one accessory component 12 representing any kind of attachment, accessory or the like, and a tank component 13 for receiving (liquid) consumables.

The high complexity of such medical devices 1 is a considerable challenge for the users of such a device. Thus, the user 2 must be properly trained before use. Further, it is desirable to monitor a user 2, when operating the device, in order to recognise a potential improper operation at an early stage. In addition, support to guide the user 2 during operation is also very beneficial.

In the medical system as shown in figure 1, an augmented reality (AR) device 3 may be used for assisting, monitoring and/or training the user 2 in connection with an operation of the medical device 1. Any kind of appropriate device which could be used for AR purposes may be used. For example, the AR device may be any kind of AR headset or AR glasses. Furthermore, any other kind of device such as a tablet computer, a smart phone, etc. which can provide AR capabilities may be possible, too.

The AR device 3 may comprise or may be in in particular (wireless) data connection with one or more output components. For example, an output component may provide optical/graphical output. Such optic/graphical output may be provided, for example on a display. The display may be a non-transparent display on which an image of the environment, in particular an image of the medical device 1 or at least part of the medical device 1, is overlaid with further graphical elements. The display may also be a semi-transparent display so that the user 2 can look through this display in order to recognize the real environment. Furthermore, additional graphical elements may be provided by the semi-transparent display so that graphical indications on the semi-transparent display may provide assistance to the user 2 during operation of the medical device 1.

The AR device 3 may also comprise one or more further output components such as a loudspeaker, headphones or another device for outputting signals, in particular e.g. acoustic signals. In this way, acoustic signals such as a sound or melody or a spoken text message may be provided to the user 2. Further to this, any other kind of appropriate output component may be also comprised by or connected to the AR device 3. For example, the AR device 3 may also comprise or be connected to an output component for providing haptic signals such as a vibration, shaking or the like.

The AR device 3 may further comprise or be in (preferably) wireless data connection to one or more sensor components for sensing the environment and/or operations of the user 3 and/or receiving user's requests or input data. For example, the AR device we may comprise one or more image sensing elements such as a camera or the like for capturing image data. The AR device 3 may also comprise any other kind of sensor components, for example a gyroscope, acceleration sensors, velocity sensors, etc. for sensing a movement of the user 2. However, any other kind of appropriate sensing component may be possible, too. For example, a sensor component may also be a scanner, in particular a scanner for reading a QR code or barcode, an RFID reader, etc. Alternatively or in addition, the one or more sensor components may be provided separately form the AR device 3, e.g. as room sensors, or sensors of mobile devices or other sensor components, which are in data connection.

Preferably, are or at least some of the output components and sensor components can be implemented in the AR devices 3. However, as mentioned above, it may be also possible to use external output components and/or input components which are not implemented in the AR device 3.

When using the medical device 1, the user can also be supported by a large language model (LLM), as known from ChatGPT, for example. The user may interact with such an LLM verbally. For example, a spoken request such as a phrase or question may be received (e.g. on a sensor component) and provided to the LLM. For this purpose, the request may be received by an input device, in particular an input device of the AR device 3, such as a microphone or the like, converted into text by a speech-to-text module and provided to the LLM. However, any other kind of input, such as entering the text by an input terminal, e.g. by means of a keyboard or the like, may be possible, too. The LLM may provide an appropriate response to the input of the user. This response may be provided to the user by an acoustically output, for example a synthetically generated voice output, by providing a text message, providing any other kinds of indication such as lines, arrows, symbols, icons, images and/or video clips. For this purpose, an appropriate output component of the AR device 3 may be used. The output may relate to output which is dynamically generated by the LLM in response to the request of the user. However, the output may also comprise, for example prestored elements which have been generated in advance. For example, the output may comprise prestored images such as images of the medical device 1 in a specific configuration, images of a specific part of the medical device 1, a video clip for illustrating a specific operation of any other kind of data which has been prepared in advance.

When generating the output, the LLM may take into account the language of the user. Accordingly, the LLM may provide the response in the same language as used by the user for formulating the request. For example, when the user asks a question in English, the LLM may also provide the response in English, and when the user asks a question in German, the LLM may provide the response in German. However, it may be also possible to limit the processing and/or the generation of the response to one or more specific languages. Furthermore, it may be also possible to provide a response of the LLM to a specific language regardless of the language in which the input was formulated. For example, the LLM may always provide a response in a language corresponding to which the medical device 1 is set.

The dialogue with the LLM may be used for any kind of interaction, in particular for any kind of assistance which can help the user in operating the medical device 1. For example, the user may ask which component or which part of the medical device 1 is currently located in front of him. For this purpose, it may be also possible to take into account supplementary information. For example, an input component, in particular input component of the AR devices 3, may detect a viewing direction of the user and/or the environment of the user, in particular it may be possible to detect/identify elements or objects in the environment of the user. Accordingly, this information may be also taken into account when the LLM generates a response to the request of the user.

The user may also ask, for example, how to perform a desired operation, for example how to attach an accessory or disposable, or how to execute a next step of the desired operation or the user may ask where a specific element of the medical device is located. In order to generate a response to such a request, it may be possible to take into account any kind of additional information such as information acquired by a sensor component, in particular a sensor component of the AR devices 3. For example, image data of the environment may be captured by an optical sensor component such as a camera. The image data may be processed in order to identify the medical device 1, the configuration of the medical device 1, accessories of the medical device, a spatial relationship between the user and the medical device 1, a current operation of the user or to detect a spatial position of a part of the body of the user, such as the current position of a hand of the user. Further, it may be also possible to receive supplementary information from any other kind of data source. For example, it may be also possible to receive information about the current configuration of the medical device 1, a current state of the medical device 1, operation parameters of the medical device 1 or any other property of the medical device 1 via a wired or wireless communication link.

Accordingly, the user may be assisted by a verbal interaction with the LLM in order to carry out a desired task and/or to get more information about the medical device 1. The information provided by the LLM do not necessarily have to refer only to a task currently being executed by the user. It is also possible for the user to interact with the LLM to obtain any other kind of information, for example general information in connection with the medical device 1.

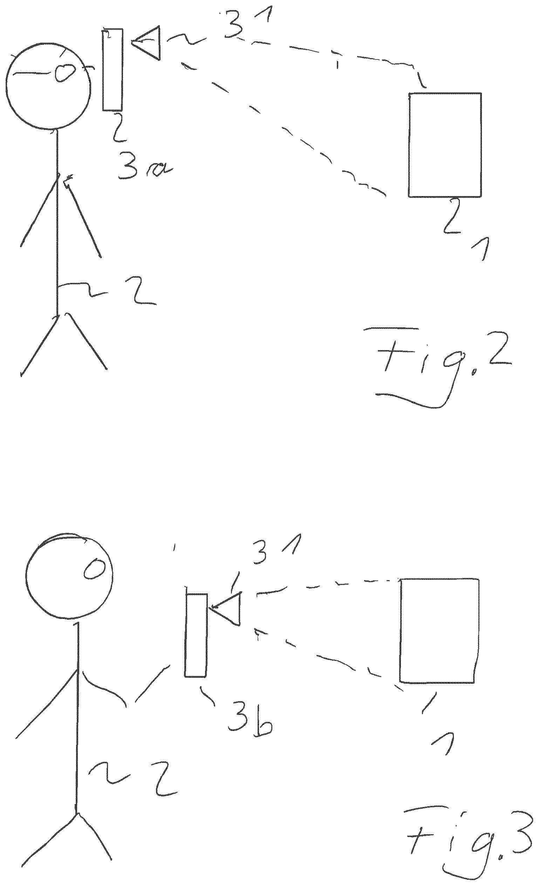

The other components, depicted in Fig., 1 are described below (after Fig. 3) in more detail.

**Figure 2** shows a schematic illustration of an AR device 3a, used when a user handles or operates the medical device 1, wherein the AR device 3a comprises a headset or AR glasses. In such an embodiment, the AR device 3a may be arranged on a head of the user 2. The user 2 may look through a semi-transparent display. Accordingly, the user 2 can still recognise the environment. In addition, graphical elements may be provided by the semi-transparent display so that the additional graphical elements may be shown at positions related to elements in the real world. In addition, the AR device 3a may also comprise further output elements such as loudspeakers or the like in order to provide acoustic output. Further, the AR device 3a may comprise a camera 31 in order to capture image data, in particular image data in a direction in which the user 2 is currently looking. Further, the AR devices 3a may comprise any other kind of sensor components for monitoring a movement of the AR device 3a and a corresponding movement of the user 2 or for acquiring any other kind of appropriate sensor data. The AR device 3a may also comprise any other kind of sensors, for example a microphone for receiving voice commands from a user. The sensor component, e.g. in the form of the camera 31 may alternatively or in addition be provided externally to the AR device 3, 3a.

**Figure 3** shows a schematic illustration of another type of AR device 3b. In this configuration, the AR device 3b may be realised by a handheld device such as a tablet computer or smartphone. The handheld AR device 3b may comprise a camera 31 for capturing image data, in particular during operation of the medical device 1. Further, the handheld AR device 3b may comprise a display for providing the captured image data in combination with additional graphical elements. In this way, the image data of the real environment may be overlaid with additional graphical elements. Alternatively, the handheld AR device 3b may comprise a semi-transparent display so that the user 2 can look through this semi-transparent display. In such a configuration, additional graphical elements may be provided on the semi-transparent display in order to provide additional information, in particular information related to one or more elements to which the user 2 is looking through the semi-transparent display. In any case, the handheld AR devices 3b may also comprise output components for providing acoustic and/or haptic output. Further, the handheld AR device 3b may comprise or be connected to any kind of sensor component, e.g. a microphone for sensing acoustic signals, in particular voice command of the user, or sensors for monitoring a movement of the handheld AR device 3b.

The AR device 3, 3a, 3b may be controlled, for example, by means of a control device 4.

In figure 1, this control device 4 is illustrated as a separate component which is arranged external to the medical device 1. However, it may be also possible to implement this control device 4 into the medical device 1. Especially, it may be even possible that the functionalities of control device 4 may be realised by components of the medical device 1.

Control device 4 may receive sensor data from one or more sensor components, in particular sensor components of the AR device 3 or external sensor components, or another device related to AR device 3. Furthermore, control device 4 may also receive further information or a data from the medical device 1. For example, control device 4 may receive data relating to the configuration of the medical device 1, a current setting of the medical device 1, data for specifying a fill level of consumable of the medical device 1, operational data such as, for example, a flow rate, a pressure value or the like, status or error messages or any other information which are related to medical device 1.

Control device 4 may also establish a communication link with one or more external storage devices, in particular one or more databases 5. For example, such a database 5 may provide any kind of information related to the medical device 1. Such information may comprise, for example, any kind of data for specifying the medical device 1. For instance, control device 4 may receive a serial number of the medical device 1 and refer to a database 5 in order to obtain further information in connection with this serial number. It may be also possible to use image data, in particular image data captured by a sensor component of the AR device 3 in order to identify the medical device 1 or an attachment/accessory of the medical device 1. For example, a QR code or barcode may be scanned and control device 4 may refer to database 5 in order to identify the medical device 1 based on this code. Alternatively, it may be also possible to identify any appropriate features in an image representing the medical device 1, and to refer to a database 5 in order to identify the medical device 1 based on these features. However, any other approach for identifying a medical device 1, an accessory or attachment, or to retrieve any other information in connection with the medical device 1 may be possible, too.

Database 5 may also provide, for example, further relevant information such as recommended settings, a fill level of consumables, predefined operations for a user for operating the medical device 1 of any other kind of appropriate information in connection with medical device 1.

Database 5 may also provide any kind of information for specifying operations in order to carry out a specific task in connection with the medical device 1. Such a specific task may be, for example, a task for configuring the medical device 1, a task for operating or monitoring the medical device 1 during operation, a task in order to resolve a warning or failure of the medical device 1, a task for maintenance of the medical device 1 or even a task for repairing the medical device 1. However, any other kind of task may be specified, too.

Database 5 may also comprise further information in connection with one or more users 2. For example, the database 5 may store and provide any kind of preferences of a user 2. Such preferences may comprise, for example, a specific configuration, in particular a specific configuration for providing information by the AR devices 3, e.g. a colour scheme, a specification how detailed a support/assistance for the respective user 2 should be, a degree of knowledge or a skill level of the respective user 2, language skills, physical limitations of the user 2, or any other data in connection with the user 2.

Further, databases 5 may also provide any kind of information in connection with a patient which shall be treated by the medical device 1. Such information may comprise, for example, a physical characterisation of the patient, a desired therapy/treatment, medication of the patient or any other relevant data in connection with the patient.

In addition, database 5 may also provide any other kind of relevant information which could be useful in connection with an operation of the medical device 1.

Even though database 5 is illustrated in figure 1 as a single database 5, it is understood, that database 5 may be also realised by multiple separate databases. For example, separate databases may be provided for the properties of the medical device 1, data in connection with the operation of the medical device 1, the data in connection with a patient, the preferences of the user, etc. The individual databases 5 may be also located at different spatial locations. Further to this, it may be also possible to store at least some of the data in a local database within the control device 4.

As can be further seen in figure 1, it may be also possible to establish a connection to a further expert, in particular a further human expert 6. Such a further human expert 6 may be located close to the user 2 and/or the medical device 1. For example, the expert 6 and the user 1 may be located in a same room. However, it may be also possible that the expert 6 is located remotely. For example, expert 6 may be located in a separate room in a same building or any other position. For example, expert 6 may be located in another city, country or even continent.

Especially if the expert 6 is located somewhere outside the room with the user 2 and the medical device 1, expert 6 may be provided with any kind of appropriate information in connection with the medical device 1. For example, expert 6 may receive information of the current status, operational properties, warnings failures etc. of the medical device 1. Furthermore, expert 6 may also receive information in connection with a desired task to be executed currently in connection with the medical device 1. Expert 6 may also receive information in connection with the user 2 and/or the patient to be treated. However, any other kind of information may be also provided to the expert 6. For example, expert 6 may receive image data captured by one or more cameras, in particular one or more cameras of AR device 3. The information may be provided to the expert 6 in any appropriate manner, for example by means of a display, such as a monitor or a virtual reality (VR) or AR device.

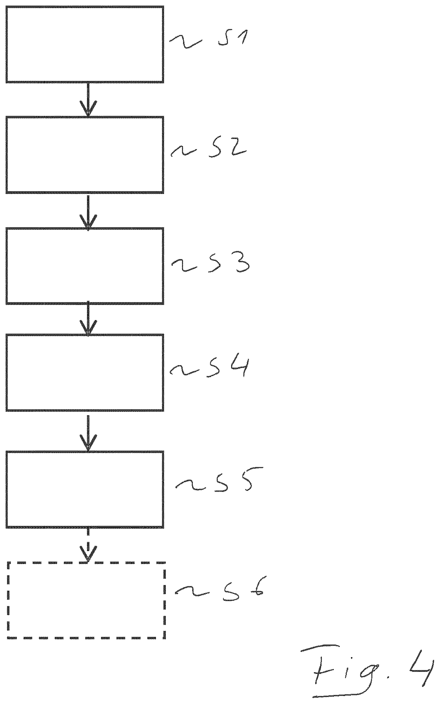

**Figure 4** shows a flow diagram for a method for assisting a user 2 on or at a dialysis device 1 according to an embodiment.

The term "on or at" refers to the fact that the assistance method may be used, when or during working with the medical device. All tasks or functions and/or actions to be performed in this respect may be supported. It is not necessarily required that the user directly operated on the medical device but may e.g. work at accessory devices, like e.g., disposables or related devices in the context of the (intended) treatment with the medical device. Further, assistance is also available in a setup or configuration phase of the medical device before the actual operation of the medical device.

Generally, the method may be implemented, for example, in the above-described medical system. Thus, the embodiments relating to systems, devices or apparatuses may comprise any kind of device, component or unit for implementing the features of the embodiments relating to a method. Accordingly, embodiments relating to the method may comprise any appropriate method steps for realising a feature as described in connection with an embodiment relating to a system, apparatus or device.

The method for assisting a user 2 on a dialysis device 1 comprises a step S1 for capturing data related with dialysis device 1. The data may be captured, for example, by at least one sensor component. The at least one sensor component may be, for example, a sensor component of an AR device 3. As already mentioned above, the sensor component may be, for example, an image capturing device such as a camera. For example, such an image capturing device may capture image data in a direction on which the user 2 is currently looking or on which the AR devices 3 is directed to. The data may be also captured, for example, by means of a microphone or the like for acquiring acoustic signals. For example, a sensor component may receive voice commands from the user 2. Further, a sensor component may read a QR code, a barcode, and RFID tag or the like. Further to this, a sensor component may be used for detecting a position and/or a movement of the user. For this purpose, appropriate sensors such as a gyroscope, an acceleration sensor or the like may be used. Furthermore, data in connection with the dialysis device 1 may be also receive, for example, by an appropriate interface such as a wired or wireless communication channel.

The data related with the dialysis device 1 may be any kind of data in connection with the dialysis device 1. For example, the data may be used in order to identify the dialysis device 1, to detect any kind of attachment or accessory of the dialysis device 1, determine a particular configuration of the dialysis device 1, identify a setting of the dialysis device 1, detect a fill level of a consumable for the dialysis device 1 or to determine any other appropriate data related to the dialysis device 1.

In a step S2, an operational setting of the dialysis device 1 may be identified. The operational setting may be identified, for example, based on captured data, in particular the data captured in step S1. The operational setting may comprise, for example identifying a configuration of the dialysis device 1 such as a configuration comprising specific attachments or accessories, a determination of a fill level of a consumable in the dialysis device 1, a specific status of the dialysis device 1 or any other property for characterising the dialysis device 1. The operational setting may be determined, for example, by processing image data captured by an image capturing sensor component. For example, an image of the dialysis device 1 may be processed in order to identify accessories or attachments, identify and evaluate control lights on the dialysis device 1, detect and evaluate a control panel, in particular an output message displayed on such a control panel, or any other appropriate operation for determining the setting of the dialysis device 1. Furthermore, it may be also possible to receive any kind of data from the dialysis device 1 via a wireless or wired communication channel in order to determine the operational setting of the dialysis device 1. It may be also possible to detect, for example, a QR code or barcode or an RFID tag on the dialysis device 1 or an attachment or accessory on the dialysis device 1 and to determine the respective attachment or accessory based on such an element.

The identification of the operational setting of the dialysis device 1 may also comprise determining a spatial position of the dialysis device 1 or any other component in connection with the dialysis device 1. For example, a relative position of the dialysis device 1 with respect to the user 2 and/or a patient may be determined by processing appropriate image data or any other kind of information. Furthermore, it may be also possible to take into account the environment of the dialysis device 1. For example, it may be possible to determine an available space surrounding the dialysis device 1. For this purpose, dimensions of a room in which the dialysis device 1 is located, may be determined, or any other features in connection with the available space and/or the spatial position of the dialysis device 1 may be determined.

In a further step S3, a task to be executed may be determined. In particular, the task to be executed may be a task which is to be executed on the dialysis device 1 by the user 2. Such a task may be, for example, a task for preparing or configuring the dialysis device 1, in particular a task for configuring the dialysis device 1 for a successive treatment of a patient.

The task may also relate to activities which shall be performed during a treatment of a patient. For example, such tasks may comprise monitoring relevant operations of the dialysis device 1, dealing with any kind of messages, warnings or failures during the treatment of the patient. Furthermore, a task may also relate to any kind of activities after the treatment of the patient has been finished, for example removing accessories, clearing the dialysis device 1 or the like. A task may also relate to maintenance operations, such as periodic maintenance operations which have to be performed after predetermined time intervals. A task may also relate to a service such as an operation for repairing the dialysis device 1 by the service person. However, any other kind of task which shall be performed in connection with the dialysis device 1 may be possible, too.

The task to be executed by the user 2 may be determined, for example, by receiving a manual input from a user 2. For example, the user 2 may enter a specification for a particular task or a selection out of a number of available tasks on a user interface or an input terminal. However, it may be also possible to determine an appropriate task in an automated manner. For example, a task to be executed may be automatically determined based on the current (identified) operational setting of the dialysis device 1. For instance, if a specific status, warning, failure or the like is identified, it is possible to determine an appropriate task in response to this. For example, if it is detected that a fill level of a consumable is below a predetermined limit, a task may be a task for filling/refilling this consumable in a tank of the dialysis device 1. If it is detected that a flow rate is below a predetermined threshold value, a task may be related to operations for overcoming this issue, for example, by cleaning or replacing a filter element or the like.

A task may be also determined automatically, for example, by referring to further information. For example, it is possible to receive information related to a patient. Such information may be received, for example, from a local or remote patient database. Accordingly, it may be possible to analyse the patient data in order to determine an appropriate treatment or a specific configuration of the dialysis device 1 taking into account the particular demands for such a patient.

A task may be also determined by any other appropriate manner, for example by a combination of an automated and manual approach. For example, it may be possible to perform a first step of analysing the dialysis device 1, the operational setting of the dialysis device 1, further features such as patient data, data in connection with the user 2 and/or any other appropriate data in order to determine a set of possible tasks. Accordingly, such a determined set of possible tasks may be provided to a user 2, for example by a user interface or the like, and the user 2 may select one of the tasks out of the provided set of tasks. For this purpose, any kind of interaction with the user 2, for example by means of a user interface or by receiving a voice command from the user 2 may be possible.

In a step S4, activities of the user 2 may be monitored. In particular, the activities of the user 2 may be monitored based on data captured by one or more sensor components such as sensor components of the AR device 3. For example, a movement of the user 2 may be monitored by appropriate sensors such as a gyroscope, acceleration sensor, etc. Furthermore, image data from one or more image sensors such as a camera may be analysed in order to evaluate the operation of the user. For example, a camera may capture images of a hand of the user 2 or another feature related to the operation of the user 2. The image data may be processed and analysed in order to identify any kind of operation performed by the user. For example, a pattern recognition, feature extraction, etc. may be performed in order to determine an operation of the user 2. It may be also possible to use a neural network or the like in order to process the image data in order to identify an operation of the user 2. Furthermore, any other kind of data may be received. For example, it may be also possible to receive data from the dialysis device 1 or another data source which could be also taken into account for monitoring the activities of the user 2.

When operating the dialysis device 1 by the user 2, the user 2 may be assisted by any kind of appropriate output, in particular by output of indications, provided on an output component such as an output component of the AR device 3. For this purpose, an appropriate assistance scheme may be determined in step S5.

An assistance scheme may define and determine the way the assistance is to be provided to the user. The assistance may e.g. be provided on different devices (e.g. output component of the AR device or of other devices), for several times (iterational modus), by different signals (acoustic, optic, and/or haptic and/or others or a combination thereof), for different time phases (only very short, as e.g. alarm tone or continuously etc.), and/or in different formats (text message, optical image etc.) etc.

The assistance as such may comprise any kind of information which could be useful for assisting the user 2 in operating the dialysis device 1 and/or for guiding the user 2 through a desired task. For example, an assistance may comprise text messages which could be provided to the user 2 on a display, in particular a display of AR device 3 or as a spoken text message by means of a loudspeaker of the like. The assistance may also comprise, for example, static or dynamic indications, which could be used to indicate a position of a specific element such as control element, an accessory, an opening for filling in a consumable, etc. The assistance may also comprise images of a specific configuration, of an attachment or accessory, how an attachment or accessory should be mounted to the dialysis device 1, or any other relevant image information. The assistance may further comprise, for example, video are sequences for illustrating how a specific task or a part of the task should/could be carried out. However, anything else, which could be useful for a user 2 when carrying out a desired task may be also comprised by such an assistance.

The assistance scheme and/or the assistance (meaning: the content of the assistance) may be determined, in particular computed, for example, by the control device 4. For example, it is possible to determine the assistance scheme and/or the assistance by an appropriate approach such as an algorithm, which takes into account the current operational configuration of the dialysis device 1. Furthermore, the task to be executed may be also taken into account for determining in the assistance scheme and/or the assistance. Additionally, the assistance scheme and/or the assistance may be determined based on any kind of information in connection with the monitoring of the activities of the user 2. Further to this, any other kind of relevant information which could be useful in this context, may be considered, too.

In order to determine the assistance scheme and/or the assistance it may be also possible to use, for example, a machine learning module, for example any kind of appropriate neural network. In this way, the neural network may receive relevant information such as the operational setting of the dialysis device, the task to be executed, the monitored activities of the user and/or further relevant information. Based on this information, the neural network can provide data for specifying an appropriate assistance scheme and/or the assistance.

For determining the assistance scheme and/or the assistance, it may be also possible to obtain additional data from a local or remote storage devices such as a database or the like. For example, the storage device may store and provide data such as text, audio sequences, graphical elements, images, which are sequences. The data may be stored in a manner which specifies a relationship between the respective data and further features such as relevant tasks, configuration of the dialysis device 1 or other relevant features. In a possible embodiment, it may be further possible that data, in particular raw data are obtained from a storage device, and the obtained data are further processed in order to adapt the data for an appropriate assistance scheme and/or the assistance or to compute an appropriate assistance scheme and/or the assistance based on the received raw data. For example, a further processing of obtained raw data may be necessary in order to adapt the assistance scheme and/or the assistance according to the current configuration of the dialysis device 1 and/or the current activities of the user 2. However, any other kind of processing of the obtained data for generating or adapting the assistance scheme and/or the assistance may be possible, too.

The assistance scheme and/or the assistance may be also adapted depending on the current user 2. For example, it may be possible to consider physical constraints or limitations of the user 2, such as a size of the user 2. For example, a small person may not reach everything which could be reached by a tall person. Furthermore, it may be also possible to consider whether the user 2 is a left-handed person or a right-handed person. However, any other property of the user 2 may be taken into account, too.

Further, may be also possible to take into account a degree of knowledge or a skill level of the user 2. For example, an unexperienced user 2 may be provided with a large amount of information and/or information having a higher level of detail. A more experienced user 2 may be provided with a small amount of information and/or information having a lower level of detail. For this purpose, the user 2 may enter his skill level manually by means of any appropriate input device. Additionally or alternatively, a skill level may be also determined fully or at least partially automatically. For example, a skill level may be determined based on the period of time, the user 2 has been already worked with a dialysis device 1 having a same or a similar type. Furthermore, the skill level may be also evaluated, for example by comparing the user's activities with related, predefined reference operations. Such reference operations may be provided, for example by any kind of local or remote storage device, for example a reference database. Accordingly, any kind of appropriate scheme for evaluating the skill level of the user 2, in particular by comparing the operations of the user 2 with related reference operations may be applied. Further, it may be also possible to specify a skill level of the user 2 by a rating of a further person, for example a trainer or another expert such as expert 6. For example, expert 6 may supervise the operations of the user 2 and give a rating based on this supervision.

The assistance scheme and/or the assistance may be also determined and/or adapted based on any kind of user preferences. For this purpose, the user 2 may enter his preferences. For example, the user 2 may specify his preferences by means of a user interface. Further, the preference of the user 2 may be also received by any kind of appropriate sensor component, in particular a sensor component of the AR devices 3. The preferences of the user 2 may specify, for example a degree of detail for the provided assistance during an operation, any kind of specifications for a graphical scheme such as a colour scheme, size of graphical elements, volume of sounds or spoken text, or any other user relevant property. The preferences of the user may be stored, for example, in a local or remote memory.

The assistance scheme and/or the assistance may be a further determined by taking into account the current activities of the user 2. For example, it may be possible to compare the current activities of the user 2 with related reference operations for carrying out a desired task. As already mentioned above, such reference operations may be obtained, for example, from a local or remote storage such as a reference database. As long as the operation of the user 2 corresponds to the related reference operations, no or only a reduced assistance may be provided to the user 2. If the operation of the user 2 is different from the related reference operations, the assistance may comprise any kind of assistance in order to perform the desired task correctly. For example, a degree of deviation between the current operation of the user 2 and the related reference operations may be determined, and the assistance may be adapted depending on this degree of deviation. If only minor deviations are detected or the detected deviations have now or no relevant impact on the result of the operation, the assistance may comprise only a small amount of assistance. For example, only text with a hint, a graphical element such as an icon or any other minor indication may be provided to the user. If, however, a larger deviation is detected or detected deviation will have significant impact on the result of the operation, the assistance may comprise any kind of element which is appropriate for drawing the user's attention to this deviation from the reference operation. For example, a larger indication may be provided on a display and/or the caustic signalling may be provided. It is understood, that several levels of deviation between the current operations of the user and the related reference operations may be taken into account for adapting the assistance and/or its scheme.

In order to determine the appropriate assistance scheme and/or the assistance, any kind of processing may be applied. For example, a rule or a set of rules may be used in order to determine, generate or select an appropriate assistance scheme. For example, one or more rules for determining the assistance scheme and/or the assistance may be obtained from a local or remote storage such as a rule database. A rule in this context may be, for example, any kind of appropriate specification for determining, selecting or generating an assistance scheme an/or its respective content which could be useful for assisting the user 2 in order to carry out a desired task or operation. A rule may specify an association of a user's assistance request and an assistance scheme and/or the assistance.

The determined assistance scheme may be used in a step S6 for providing appropriate indications to the user when executing the dialysis device. In particular, appropriate indications may be provided to the user by means of an output component, especially in output component of the AR device 3.

For example, a graphical indication may be provided to the user 2 in order to indicate a spatial position of a relevant element of the dialysis device 1 of the related component, which needs to be activated or operated next. For example, an indication may be provided on a display in order to indicate a position of the relevant control element, a position for filling a consumable, a position for attaching an accessory or attachment, etc.

The indication may be a static indication for indicating a specific position or a dynamic indication. A dynamic indication may be used, for example, in order to indicate a movement of the control element, for example for moving a switch in a desired position or for rotating controller. The indication may be provided, for example, by means of a semi-transparent display, wherein the provided graphical indication is displayed on a position so that the user will recognise the relevant position when looking through this semi-transparent display. Alternatively, it is also possible to capture an image of the environment by means of a camera and to overlay the graphical element and the captured image data on a display.

When providing, for example, a sound or video sequence, a speed of such a sequence may be adapted depending on the speed of an operation which is carried out by the user. In this way, it is possible to achieve good correlation between the provided indication and the operation of the user 2.

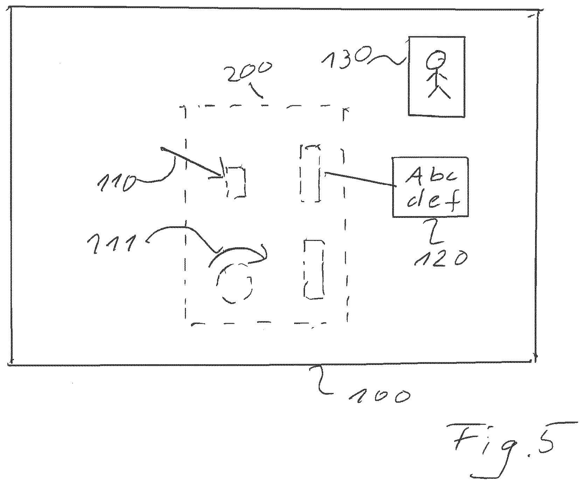

**Figure 5** shows a schematic representation of a scene 100 which may be visible when using an AR device 3 during an operation or configuration or setup of a dialysis device 1. In the representation of figure 5, the dialysis device 1 is illustrated by an object 200 represented by dashed lines. The dashed lines are to represent objects that are present in the real environment. Such objects may be perceived by looking through a semi-transparent display.

Alternatively, it may be possible to capture image data of the environment and to display the image data on a non-transparent display.

As can be further seen in figure 5, additional elements 110, 111, 120 and 130 may be provided. It should be noted that these elements 110, 111, 120 and 130 are only for illustrative purposes and do not limit the scope of the present invention. Moreover any other kind of graphical representation and any other number of graphical elements may be possible, too.

For example, a line or an arrow 110 may be used as an element for assisting the user. Such a line or arrow 110 could be used to indicate a position, in particular a position at the dialysis device 1. Furthermore, an arrow 111 of another appropriate graphical element may be used for indicating a direction of movement of a control element such as a switch, knob, rotary controller or the like.

As further illustrated in figure 5, any kind of text or other reasonable information may be provided, for example, by means of a text box 120 of the like.

The indications for assisting a user may also comprise any other kind of representation such as graphical elements, in particular geometrical elements like a circle, rectangle or the like, icons, images, etc. The indications may comprise static elements or dynamic elements. For example, a dynamic element may be any kind of graphical element for illustrating, e.g., a direction of movement or a sequence of an operation.

The indications may be provided in association with the dialysis device 1 respectively the representation 200 of the dialysis device 1, as illustrated, for example by elements 110, 111 or 120. Furthermore, it may be also possible to provide any kind of indication without a (direct) relation to the dialysis device 1, as illustrated, for example by indication 130. Such an indication may be provided, for example at a predetermined position, for example in an upper or lower area, or a predetermined area, in particular corner of the display used for providing the indications. However, it may be also possible to dynamically adapt the position for providing an indication 110, 111, 120 or 130. For example, a position of an indication 110, 111, 120 or 130 may be automatically determined in such a manner that the indication does not or only minimally covers the representation 200 of the dialysis device 1. Furthermore, it may be possible to determine relevant elements or positions of the dialysis device 1 and to adapt the position of the indications 110, 111, 120 or 130 so that the relevant elements are not only minimally covered. However, any other appropriate approach for adapting a position, size and/or a style for providing the representation may be possible, too. For example, a representation an indication 110, 111, 120 or 130 may be provided as a solid representation, if the representation does not cover a relevant element of the dialysis device 1. In case that the representation of an indication 110, 111, 120 or 130 would cover a relevant part of the dialysis device 1, the respective representation of an indication 110, 111, 120 or 130 may be provided in a semi-transparent style. Alternatively, a size of the respective representation for the indication 110, 111, 120 or 130 may be adapted, in particular reduced. However, any other appropriate scheme for adapting a property of a representation of an indication 110, 111, 120 or 130 may be possible, too.

In order to further assist the user 2 during operation of the medical device 1, the user 2 may receive further information from an artificial or human expert 6, or the user may even interact with such an artificial or human expert 6.

For example, an artificial assistance may be computed in an automated manner. The output of such an artificial assistance may be similar to an assistance of a human expert, wherein the output, however, is generated by an appropriate computing device. For example, the desired task to be executed, the monitored activities of the user 2, any kind of appropriate information from the dialysis device 1, and if available, further information may be taken into account in order to determine appropriate assistance which can be provided to the user 2 with information of an expert. Such information can be provided to the user to inform of a (virtual) expert. It may be possible that the user 2 formulate his requirements or needs in any appropriate manner, and an automated response to this request is generated and provided to the user 2 inform of an answer or hints from an expert. For example, the user 2 may express his needs inform of spoken text which are analysed and processed by speech recognition. However, any other scheme, for example specific gestures or manual input may be possible, too.

Alternatively, the user 2 may be connected to a human expert 6 (e.g., by means of digital and computer-implemented means, like digital meeting platforms). The human expert 6 may be located in a same room as the user 2 and the dialysis device 1. However, the human expert 6 may be also located spatially separated in a different room or even in a different location (city, country etc.). In particular, the human expert 6 may be located in a different room of the same building, human expert 6 may be located in a different building, even in a different city, a different country or even in a different continent. The human expert 6 may be connected with the user 2 and/or the control device 4 by means of any appropriate communication connection. For example, human expert 6 may be connected via the Internet, in particular via a secure communication channel such as a VPN tunnel.

The human expert 6 may be provided with any kind of relevant information, such as information in connection with the dialysis device 1. For example, the human expert 6 may be provided with the identified operational setting of the dialysis device 1. Further, human expert 6 may also be provided with information comprising a current status of the dialysis device 1, for example, a current flow rate, fill level of consumables, status or error messages or any other relevant information in connection with the dialysis device 1.

The human expert 6 may be further provided with additional relevant information such as the task to be executed, information with regard to the patient, constrains of the treatment location, etc. For example, such information may comprise information of available accessories, attachments, consumables or the like.

Further, human expert 6 may be also provided with information with regard to the user 2. For example, human expert 6 may be provided with information with regards to a knowledge or skill level of the user 2, or any other further needs, requirements or wishes of the user 2. Such information may be specified, for example, by the user 2 in advance and/or may be obtained from an appropriate user database.

For communication between the human expert 6 and the user 2, any kind of appropriate communication channels may be established. Such a communication channel may be used, for example, for voice or video communication. In addition, the human expert 6 may specify further assistance which could be provided to the user 2 by means of an output component, in particular the output component of AR devices 3. In this way, the human expert 6 may provide the user 2 with graphical indications to a specific position at the dialysis device 1 or hints for operating the dialysis device 1. Such indications may be provided to the user 2 in a same or similar manner as the indications of the assistance scheme.

If a same or similar dialysis device is available at the location of the human expert 6, it may be also possible to capture static or dynamic images of this device and transmit the image data to the user 2 so that the image data can be displayed on a display of the user 2, e.g. by means of the AR device 3.

In order to provide the human expert 6 with further information, any kind of information captured by the sensor components at the location of the user 2, in particular by the sensor components of AR device 3 may be transmitted to the human expert 6. For example, human expert 6 may use a VR and'/or AR device for viewing/analysing the data from user 2 and/or the dialysis device 1. In this way, the human expert 6 is provided with a virtual environment which is very similar to the configuration at the premises of the user 2.

The human expert 6 can be connected to the user 2 right from the beginning of operating the dialysis device 1. Alternatively, it may be also possible that a human expert 6 is only connected upon request or at predefined time intervals (e.g., every 10 minutes) or on an event-basis (in a preparation phase, the events for triggering the connection of the human expert may be defined, based on certain events, like failure state, operations which last too long etc). For example, the user 2 may request assistance of a human expert 6 in case of specific needs. For instance, the user 2 may request a connection to a human expert 6 in case of a particular event such as a failure or a problem in connection with the dialysis device 1. For this purpose, the connection to the human expert 6 may be requested by any kind of user action such as a voice command, a predefined gesture, or any other kind of user input. Alternatively or in addition to this, a connection to a human expert 6 may be also established automatically in case of a particular event, problem, failure or any other trigger issue.

In order to obtain assistance from a human expert 6, the user 2 may specify or select a desired human expert manually. For example, the user 2 may specify a human expert 6 by an appropriate input, for example an input on a user interface or the like. For example, the user 2 may select a human expert 6 out of a list of available human experts. Such a list may be a static list of available human experts or a list which is dynamically modified by taking into account a number of human experts depending on a current status of the individual experts. However, any other appropriate scheme for specifying or selecting the human expert 6 may be possible, too.

Alternatively or in addition, a human expert may be assigned to a particular medical device 1 and/or user, operating the medical device by means of an assignment algorithm. The assignment algorithm is configured to provide a mapping or matching between the human experts and the medical device 1 and/or the user, requesting such an assistance. The assignment algorithm may be based on pre-definable matching criteria. The matching criteria may be selected from the group consisting of:
- availability of the human expert in relation to the time phase, when assistance is needed or requested,
- geographical position, in particular geographical proximity of the medical device for which assistance is requested and the human expert,
- skill level of the human expert and/or knowledge for the particular medical device, for which assistance has been requested,
- historical matching data, so that the human requesting assistance and the expert are known to each other, and
- user defined matching criteria.

In order to identify and determine appropriate human experts, multiple approaches may be taken into account, in particular, an identification algorithm may be executed, which is configured to select or determine a particular human expert out of a pool or set of human experts. For example, individual demands and/or constrains for the user 2, the dialysis device 1, the desired task to be executed and/or demands of the patient to be treated, etc. is analysed by the identification algorithm. Accordingly, all relevant information, which shall be taken into account for identifying one or more appropriate human expert 6, may be received, for example read from appropriate storage components, and processed in order to match the demands with corresponding properties of available human experts.

For such an identification of an appropriate human expert, the required information relating to the human experts have to be acquired and provided to the identification algorithm. The identification algorithm serves to determine an appropriate human expert for providing the assistance via at least one AR device. For this purpose, the human experts may enter relevant information manually, for example by means of an appropriate input terminal, user interface or the like. However, it may be also possible to acquire all of at least part of the relevant information with regard to the human experts in an automated manner. For example, time periods in which an expert is available, may be determined by referring to an electronic calendar of the respective expert. For example, it may be possible to establish a data connection to a database or another storage device for the calendar data of an expert, access a calendar of personal digital assistant such as a smartphone, or any other database or storage device for the respective information. Furthermore, the availability of the expert 6 may be also detected, for example by detecting that the expert 6 is currently logged in or registered in a technical system, in particular a system which may be used for connecting the human expert 6 with the user 2. However, any other approach for a fully or partially automated manner in order to determine the availability of an expert may be possible, too. For example, it may be also possible to take into account the usual working hours of a human expert in a country where the expert currently is located.

Further to this, it may be also possible to specify preferences of the user for a desired expert or a group of experts. Such preferences may be entered, for example, by the user 2. For example, a user 2 may specify an assessment or evaluation of the human expert 6 after a session with the respective human expert 6 has been finished. Accordingly, a ranking of human experts may be generated and/or preferences of a particular user 2 for one or more human experts may be also taken into account by the assignment algorithm.

When matching the demands of a user 2 with the features of available experts, it may be also possible for the assignment algorithm to take into account the skills of the experts. Such skills may comprise, for example, language skills, skills in connection with particular diseases or treatments, skills in connection with particular medical devices, e.g. in connection with a particular type of a dialysis device or any other relevant skill. For example, such skills may be determined by receiving a manual input of the respective expert. However, at least some skills may be also determined in an automated manner. For example, language skills may be determined based on a setting of a technical system the expert currently is using, or as skill level in connection with a particular device may be determined based on the fact that such a device is available at the premises where the expert is currently located. However, any other scheme for identifying one or more properties of human expert may be possible, too. The relevant properties or features of human expert may be determined in advance and stored in an appropriate storage device, such as an expert database. Thus, when identifying one or more human experts, who match to the demands of a user 2, the appropriate expert can be identified automatically by referring to this expert database, which may be accessed by the assignment algorithm.

In order to identify an appropriate human expert 6, for example for matching the demands of a user 2 with the features of available human expert 6, a selection scheme, which may be based on pre-stored rules, may be applied. In particular, it may be possible to specify one or more selection schemes and/or rules in appropriate storage medium such as a selection database and to refer to this storage medium when applying a selection of an appropriate human expert 6 for a user 2. In this way, the identification of appropriate human expert 6 can be simplified and accelerated by applying technical assistance for selecting the human expert. Accordingly, the user 2 can be connected to an appropriate human expert 6 with in a very short period of time. Thus, user 2 will receive appropriate assistance very soon, if necessary. Thus, the user 2 can be guided through the necessary tasks for resolving problems very fast. In this way health disadvantages during a treatment of the patient can be avoided.

Alternatively or in addition the method may comprise:
- storing the determined personal information in an expert database; and
- determining demands of the user for operating the medical device. The determined demands may be stored in a demand storage.

The assigning of the human expert to a requesting user and/or a medical device, the user is operating, may be based on a matching between information stored in the expert database and the determined demands of the user.

Summarising, the present invention relates to an assistance of a user when dealing with a medical device such as a dialysis device. For this purpose, it is proposed to provide the user with assistance by means of an augmented reality device. The assistance may be provided by analysing a current situation and the needs of the user. An operational setting of the dialysis device is detected for providing automated assistance to a user. In further embodiments, the user can be assisted by an expert, wherein the user can be assisted for a full or partial automated selection of an appropriate expert.

## Claims

1. Method for providing assistance on an augmented reality, AR, device (3) for operating a medical device, in particular a dialysis device (1), comprising:
- capturing (S1) data by at least one sensor component;
- identifying (S2) an operational setting of the medical device based on the data captured by the at least one sensor component;
- determining (S3) a task to be executed on the medical device;
- monitoring (S4) activities of a user (2) based on at least the data captured by the at least one sensor component;
- determining (S5) an assistance scheme for providing assistance for carrying out the determined task based on the identified operational setting of the medical device, the determined task to be executed and the monitored activities of the user (2);
- providing assistance according to the determined assistance scheme comprising the output of indications on an output component of the AR device (3).

2. The method according to claim 1, wherein capturing (S1) data related with the medical device comprises capturing an image, scanning an optical label and/or an RF tag of the medical device and/or an accessory component of the medical device by the sensor component; and/or
wherein identifying (S2) the operational setting of the medical device comprises identifying the medical device, detecting at least one accessory component of the medical device, and/or determining a spatial position and/or a spatial orientation of the medical device.

3. The method according preceding claim 1 or 2, comprising
obtaining, from a reference database, at least one related reference operation for the determined task;
wherein the assistance scheme and/or the assistance is determined based on a comparison between the monitored activities of the user (2) and a related reference operation obtained from the reference database.

4. The method according any of preceding claims 1 to 3, wherein the task is a task to be executed by the user (2), comprising a task for configuring or operating the medical device, for monitoring the medical device during operation, and/or a task for maintenance of the medical device and/or a task for a repair or service of the medical device.

5. The method according any of preceding claims 1 to 4, wherein the assistance scheme and/or the assistance is determined based on a preset or predetermined skill level of the user (2) and/or a rule, in particular a prestored rule obtained from a rule data base.

6. The method according any of preceding claims 1 to 5, comprising a step of computing an evaluation of a skill level of the user (2) based on the monitored activities of the user (2) and the related reference operation for the determined task.

7. The method according any of preceding claims 3 to 6, wherein the assistance scheme and/or the assistance is determined depending on a difference between the monitored activities of the user (2) and the related reference operation, in particular wherein the indications are provided on the output component only if a difference between the monitored activities of the user (2) and a related reference operation is determined, being higher than a predefined threshold value.

8. The method according any of preceding claims 1 to 7, comprising a step of providing real-time support for operating the device from an artificial expert and/or human expert (6),
wherein the real-time support is provided on the output component of the AR device (3).

9. The method according preceding claim 8, comprising a step of receiving a user request for providing real-time support, by at least one sensor component,
wherein providing the real-time support is controlled based on parameters of the received user request.

10. The method according preceding claim 8 or 9, wherein a human expert is determined out of a set of human experts (6) by executing an identification algorithm and/or wherein the human expert (6) is assigned to a particular user (2) or to a particular medical device by executing an assignment algorithm.

11. The method according to preceding claim 10, wherein the identification algorithm and/or the assignment algorithm process personal information related to a human expert, wherein such personal information is determined by:
- receiving input from the human expert,
- analysing data, in particular scheduling data, from an electronic device of the human expert,
- detecting a presence of the human expert at a predetermined spatial location,
- receiving a rating of the human expert according to an input of the user, and/or
- receiving information about the human expert, the information comprising skills, preferences, scheduling data, a rating and/or further data related with the respective human expert;
and /or wherein the method may comprise:
- storing the determined personal information in an expert database;
- determining demands of the user (2) for operating the medical device;
wherein the assignment algorithm is configured for assigning the human expert (6) based on a matching between information stored in the expert database and the determined demands of the user (2).

12. The method according preceding claim 11, wherein the demands of the user (2) are determined based on at least one of user input, in particular user input received by the sensor component, input data received from the medical device and/or patient information, in particular patient data obtained from a patient database.

13. The method according any of preceding claims 8 to 12, comprising storing monitored activities of a user (2), wherein the human expert (6) is identified based on previously stored monitored activities of the user (2).

14. The method according any of preceding claims 8 to 13, wherein the human expert (6) is selected based on a machine learning module

15. Medical system, which is configured for providing assistance on an augmented reality, AR, device (3) and which is configured to execute a method according to any of the preceding method claims, comprising:
- a medical device, particularly a dialysis device (1);
- a sensor component configured to capture data;
- the AR device (3) with an output component, and
- a control device (4), configured to
- identify an operational setting of the medical device based on the data captured by at least one sensor component,
- determine a task to be executed by the user (2) on the medical device,
- monitor activities of a user (2) based on the data captured by at the least sensor component, and
- determine an assistance scheme for providing assistance to the user (2) for carrying out the determined task based on the identified operational setting of the medical device, the determined task to be executed and the monitored activities of the user (2),
- providing assistance according to the determined assistance scheme comprises the output of indications on an output component of the AR device (3).

16. Augmented reality, AR, device (3) for use in a medical system according to claim 15, comprising:
an output device configured to provide indications based on the determined assistance scheme, when the medical device, in particular a dialysis device (1) is operated.

17. AR device (3) according to claim 16, comprising a sensor component configured to capture data related with the medical device, in particular a dialysis device (1).
